# EUROPEAN PATENT SPECIFICATION

(11) **EP 2 298 276 B1**
(45) Date of publication and mention of the grant of the patent: **15.01.2020**
(21) Application number: 09011120.4
(22) Date of filing: 13.02.2007
(51) Int. Cl.: A61P 19/10, A61P 3/06

(54) **Accelerating agent of calcium absorption**
Beschleunigungsagens für die Kalziumabsorption
Agent d'accélération de l'absorption du calcium

(30) Priority: 17.02.2006 JP 2006041158; 17.02.2006 US 774374 P
(43) Date of publication of application: 23.03.2011
(62) Divisional of application: 07714433.5
(73) Proprietor: Viridis Pharmaceutical Limited, Road Town Tortola (VG)
(72) Inventor: Matsuda, Hideaki, Habikino-shi, Osaka 5830872 (JP); Iwaki, Masahiro, Nara-shi, Nara 6308244 (JP); Kawase, Atsushi, Osaka-shi, Osaka 5360021 (JP)
(74) Representative: Hasler, Erich

(56) References cited:
- DE-A1- 10 127 897
- US-A- 5 545 414
- US-A1- 2004 101 597
- US-A1- 2007 003 671
- MEE K A ET AL: "APPLE FIBER AND GUM ARABIC LOWERS TOTAL AND LOW-DENSITY LIPOPROTEINCHOLESTEROL LEVELS IN MEN WITH MILD HYPERCHOLESTEROLEMIA", JOURNAL OF THE AMERICAN DIETETIC ASSOCIATION, THE ASSOCIATION, CHICAGO, IL, US, vol. 97, no. 4, 1 April 1997 (1997-04-01), pages 422-424, XP000676803, ISSN: 0002-8223

## Description

### TECHNICAL FIELD

The present invention relates to an agent for promoting calcium absorption.

### BACKGROUND ART

A level of calcium within the living body is constantly maintained by balancing between absorption and excretion from the gastrointestinal tract, and renal tubular reabsorption and excretion, respectively. Consequently, calcium deficiency is one of the symptoms of chronic renal failure. Also, calcium deficiency is often found since it is not easy to find various foods containing a lot of calcium, and an efficiency of the absorption is not sufficient in many cases. Calcium deficiency is responsible for brittle bones and may cause osteoporosis and osteomalacia, and thus it is hoped to establish a method for improving an efficiency of the calcium absorption from the small intestine and/or bone absorption of calcium.

As maintaining a balanced blood calcium is essential for good cardiac function, calcium deficiency has also been linked to cardiovascular disorders such as atherosclerosis, coronary artery disease, ischemic heart disease, hyperlipidemia and hypertension. Calcium has also been found to lower cholesterol levels.

Other conditions which may benefit from improved calcium absorption are malaria, ulcerative colitis, gingivitis or dental plaque, and learning defects such as Alzheimer's or senile dementia.

GA is a gummy exudation obtained from the stems and/or branch or Acacia senegal (Leguminosae) or other Acacia species (Leguminosae). The major component of GA is arabic acid (79 to 81%), which exists as Ca, Mg and/or K salts. Acid hydrolysis of GA yields L-arabinose, D-galactose, L-rhamnose, and D-glucuronic acid. In addition, traces of hydrolase and oxidase are present, together with a little amount of mineral and proteins in GA.

From the era of ancient Egypt, GA has been used as a folk medicine for the treatment of periodontal disease and alveolar pyorrhea (treating a bleeding from the gums, removing ulcers in the gums, or promoting a growth of the teeth); lung disorders and liver disorders.

At present, GA is used as an emulsifier with flavour for keeping homogeneity of ingredients of juices and ice-creams, a food additive for maintaining a shape of candies and stabilizers of medicines for compressing tablets or preventing disproportionation of the ingredients in a liquid formulation.

GA is a dietary fiber, which is difficult to digest by digestive enzymes, and is generally known to prevent calcium absorption from the gastrointestinal tract. Tokkyo Kokai H09-67257(1997) discloses an attempt to give a promoting effect on mineral absorption to chitin by reducing its molecular weight, since chitin with a larger molecular weight, prevents absorption of minerals such as calcium, magnesium and the like. Also a method for improving calcium absorption by adding lactose and sugar-alcohol to dietary fiber is proposed (Tokkyo Kokai 2002-142721).

With respect to GA, an accelerating effect on absorption of water and sodium ion from the small intestine was disclosed, but an accelerating effect of calcium absorption from the small intestine or bone absorption has not been reported.

US 2004/0101597 A1 is directed to calcium-fortified acidic beverages that are more stable against calcium precipitation and it mentions gum arabic as viscosity modifier or bodying agent.

US 5,545,414 discloses a nutritional product with dietary fiber that may be used for reducing the serum cholesterol in a mammal. Gum arabic is described as moderately useful for lowering cholesterol levels.

DE 10127897 A1 is concerned with pharmaceutical compositions containing a calcium salt and a dry plant extract. Gum arabic is used in one example as an excipient.

Mee K. A. et al: "Apple fiber and gum arabic lowers total and low-density lipoprotein cholesterol levels in men with mild hypercholesterolemia", Journal of the American Dietetic Association, Chicago, IL, US, vol.97, no.4, 1 April 1997 (1997-04-01), p.422-424, also relates to the lowering of cholesterol levels and describes the use of gum arabic for this purpose.

### DISCLOSURE OF INVENTION

Dietary fiber is believed to be one of the most important nutrients, since it prevents a rapid increase of blood glucose after a meal by controlling a rate of glucose uptake from the gastrointestinal tract, and avoids constipation with the associated excretion of harmful substances in the intestine.

The present invention provides an accelerating agent of calcium absorption, which can be taken daily, optionally with a dietary fiber, to avoid a calcium deficiency. The inventors have unexpectedly found an accelerating effect of GA on calcium absorption from the gastrointestinal tract.

According to a first aspect of the present invention, an accelerating agent of calcium absorption comprising Gum Arabic according to claim 1 is provided.

According to a second aspect of the present invention, there is a calcium-enriched food comprising a calcium agent and the accelerating agent as defined above.

Disclosed is also the use of the accelerating agent as defined above, in the manufacture of a calcium-enriched food.

According to a third aspect of the present invention, there is a drink agent to compensate for the lack of calcium comprising the agent as defined above.

Further disclosed is a composition that comprises Gum Arabic and a calcium agent, as a combined preparation for simultaneous, separate or sequential use in therapy.

According to a fourth aspect of the present invention, there is the use of Gum Arabic in the manufacture of an agent for the treatment or prevention of a disease associated with a calcium deficiency.

Disclosed is also the use of an accelerating agent as defined above, for the manufacture of a medicament for the treatment or prevention of chronic renal failure, or a calcium deficiency associated with chronic renal failure.

Additional information concerns the use of an accelerating agent as defined above, in the manufacture of a medicament for the treatment or prevention of cardiovascular disorder, including one or more of atherosclerosis, coronary artery disease, ischemic heart disease, hyperlipidemia and hypertension.

According to a fifth aspect of the present invention, there is the use of an accelerating agent as defined above, in the manufacture of an agent for the treatment or reduction of cholesterol in a hypercholesterolemic subject.

In addition, the disclosure contained in this document encompasses the use of an accelerating agent as defined above, in the manufacture of a medicament for the treatment or prevention of malaria.

The use of an accelerating agent as defined above, in the manufacture of a medicament for the treatment or prevention of ulcerative colitis, is a further part of the teaching included in this document.

Disclosed is also the use of an accelerating agent as defined above, in the manufacture of a medicament for the treatment or prevention of gingivitis or dental plaque.

Furthermore, an interesting aspect is the use of an accelerating agent as defined above, in the manufacture of a medicament for the treatment or prevention of learning defects such as Alzheimer's disease or senile dementia.

Calcium deficiency is responsible for brittle bones and may cause osteoporosis and osteomalacia. The present invention makes it possible to improve the efficiency of calcium absorption from the small intestine and/or bone absorption of calcium and to prevent the above diseases, while simultaneously taking dietary fiber which is one of the important nutrients. Therefore, the use of Gum Arabic for the manufacture of a medicament for the treatment or prevention of osteoporosis and/or osteomalacia is another interesting aspect. The medicament may also include a calcium supplement/agent.

### BRIEF DESCRIPTION OF DRAWINGS

Figure 1 shows ratios of calcium content remained in the recovered perfusion solution. Perfusion solution containing calcium (1 mg/mL) or calcium and GA (7.5%) was flowed through the tract and each sample of 500 µL was recovered at 10, 20, 30, 40, 50 and 60 min intervals after the start of the perfusion.
Figure 2 shows total urinary excretion of calcium for three days. Water containing calcium, GA or calcium + GA was administered to rats and the results were expressed by +Ca, +GA and +[Ca + GA], respectively.
Figure 3 shows the effects of feeding calcium and/or GA on body weight increase in rats. "Control" and "Ca free" means body weights of the group fed a normal diet and a calcium-deficient diet respectively.
Figure 4 shows the effects of feeding calcium and/or GA on hardness of the femur in rats. "Control" and "Ca free" are the same as described above.
Figure 5 shows the effects of feeding calcium and/or GA on the contents of Ca, Zn, Mg and P of the femur in rats. "Control" and Ca free" are the same as described above.
Figure 6 shows the effects of feeding calcium and/or GA on ALP in blood of rats. "Control" and "Ca free" are the same as described above.

### BEST MODE FOR CARRYING OUT THE INVENTION

As the water-soluble gum in this invention, a gummy exudation from the stems and/or branch of Acacia species (Leguminosae) such as Acacia Senegal and Acacia seyal may be used in its intact form. From the view point of easy availability and easy formulation, the dried powder of the said gum and/or the extract of the said gum are preferable.

The preferred extraction solvent is water. The aqueous extract can be used as it is, or after concentration, dilution and/or purification.

The dried powder and/or the extract can be purified by means of column chromatography.

The accelerating agent of the present invention can be used by mixing it with a calcium-containing food, for example, a dairy product such as milk, yogurt, cheese and the like, and by adding a calcium agent in a case of calcium-deficient or no calcium-containing food. The calcium agent used in such case includes, but not limited to, a food additive, comprising a calcium salt such as calcium carbonate, calcium lactate, calcium gluconate, calcium acetate, calcium citrate, calcium phosphate, calcium chloride and calcium hydroxide; and natural products such as shell meal, coral powder, egg shell, milk, cow bone powder.

In order to compensate for the lack of calcium, the accelerating agent of the present invention can be used by mixing it with various foods and drinks (calcium-enriched foods) as well as by combining it with a calcium agent described above. The calcium-enriched food includes, is not limited to, carbonated drinks, fruit juice, fermented drinks, milky drinks such as milk; frozen desserts such as ice-cream, ice milk; dairy products such as yogurt, cheese; luncheon meats such as ham, sausage; fish paste products; bread hotcake, prepared dish, cream caramel, soup and the like.

If necessary, an appropriate amount of sweetener such as sugar, honey, glycerine and aspartame, spice such as garlic and ginger, perfume such as fruity flavour, antioxidant such as ethylenediamine disodium salt and sodium thiosulfate, amino acid such as leucine, methionine, lysine, and taurine, and vitamin such as vitamin A, vitamin B, vitamin C, vitamin D, vitamin E, and nicotinamide, can be added to the said drink formulation.

Natural juice such as orange juice, grapefruit juice and vegetable juice (kale, young leaves or barley), and/or the extract of crude drug such as Ginseng and Young Deer Horn also can be added to the said drink formulation for oral administration.

The accelerating agent of the present invention is also available for animals other than humans, and may be used as a pet food such as dog food or cat food, and animal feed.

As described above, the accelerating agent of the present invention may be taken orally by itself or together with other foods or drinks. The amount of the agent to be taken depends on the formulation of each food or drink; and sex, age and body weight of a person taking the agent, but usually 1-100 g/day, preferably 10-50 g/day, and more preferably 10-20 g/day. The agent can be taken in various formulations, after it is dissolved or suspended in cold water, hot water or alcohol.

### EXAMPLES

The effect of GA on calcium absorption from the intestinal tract was assayed according to the following methods.

In each test, purified GA obtained from SANKYO Foods Industry Corp. and calcium L-lactate (Sigma) were used, the latter was known to have high solubility (9600 mg/100 g H2O) and no effect on the taste of food. Male Wistar rats weighing 200-250 g, and about 120 g especially in the experiment of studying calcium born absorption (Test Example 3), were used as an experimental animal.

### Test Example 1 - Study of absorption efficiency from the small intestine using a perfusion method (in situ)

### 1. Method

The abdominal cavity of the rat anesthetized with pentobarbital (50 mg/mL/kg, i.p.) was opened and a silicon tube was inserted into the duodenum and the appendix. A perfusion solution containing calcium (1 mg/mL), or calcium and GA (7.5%) was flowed through the intestinal tract, the perfusing solution was recorded over time, and the calcium content was assayed.

### 2. Result

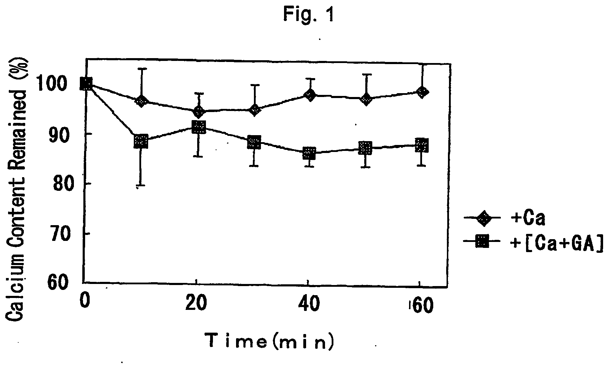

The result is shown in Figure 1. It was suggested that GA increased the efficiency of calcium absorption from the small intestine.

### Test Example 2 - Effects on Urinary excretion

Usually, calcium concentrations in blood are constantly maintained by biogenic homeostasis and it is difficult to utilize it as an index of calcium absorption. Accordingly, the urinary excretion of calcium was assayed in place of it.

### 1. Method

Rats were kept in a metabolic cage and 40 mL of water containing calcium (Ca), Gum Arabic (GA), or Ca and GA (Ca + GA) was given for three days. Furthermore, they were fed a calcium-deficient diet in order to eliminate an effect of calcium contained in the normal diet. After recovering urine, urinary concentration of calcium and urine volume were measured.

### 2. Results

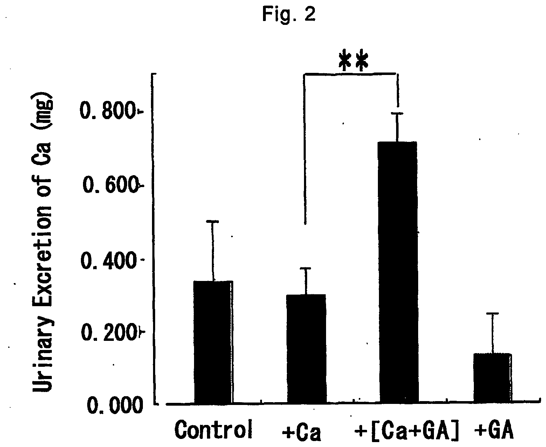

The result was shown in Figure 2. Urine was recovered for three days using a metabolic cage and urinary excretion of calcium was assayed. Urinary excretion of calcium was not increased in the Ca- and GA-treated groups, while urinary excretion in the (Ca + GA)-treated groups was significantly increased. Accordingly, it was also suggested in vivo that GA-uptake increased the efficiency of calcium absorption.

### Test Example 3 Effects on bone absorption of calcium

Next, it was studied whether the accelerating effect of GA on calcium absorption reaches bone absorption, and prevents the decease of calcium content in the bone, using rats fed a calcium-deficient diet.

### 1. Method

Calcium (Ca-treated group), or calcium and GA ([Ca + GA]-treated group) were orally administered to rats for 30 days with an amount of 1 mg(ca)/day, and changes of hardness and mineral (Ca, Mg, P, Zn) content of the femur, or ALP in blood were observed. Rats were fed a calcium-deficient diet in order to eliminate an effect of calcium contained in the normal diet.

### 2. Results

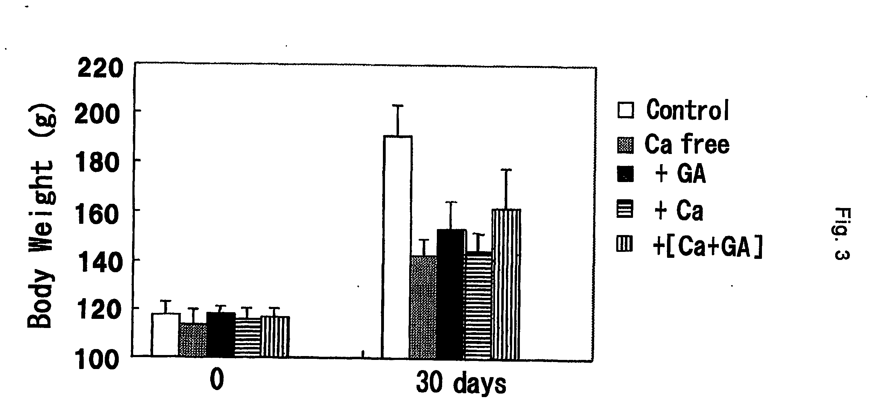

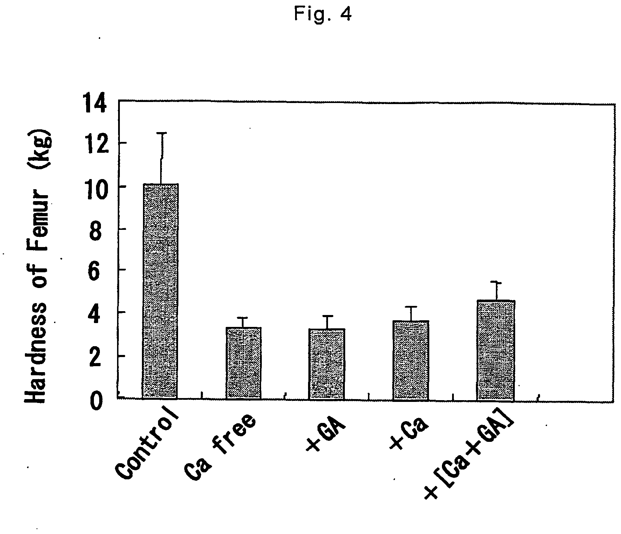

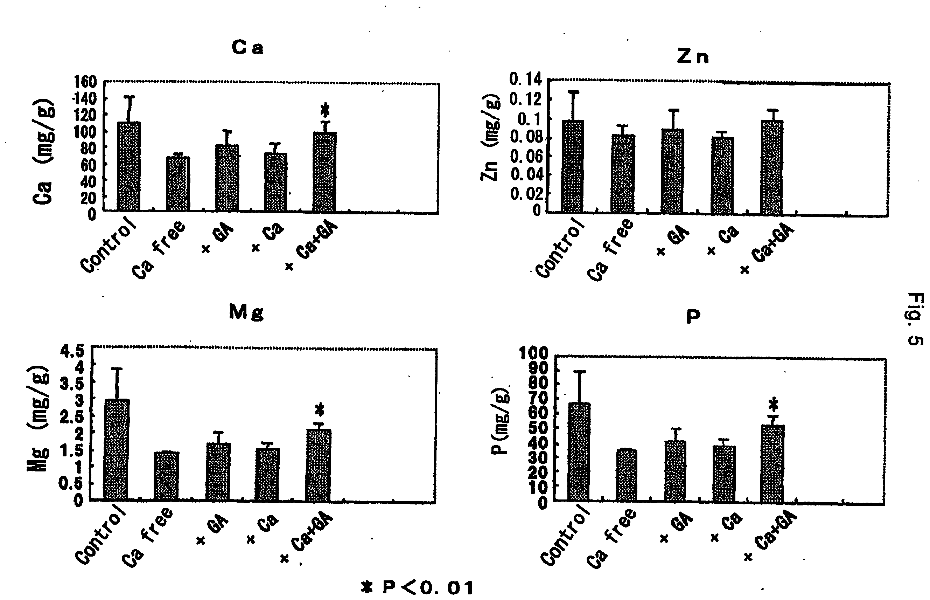

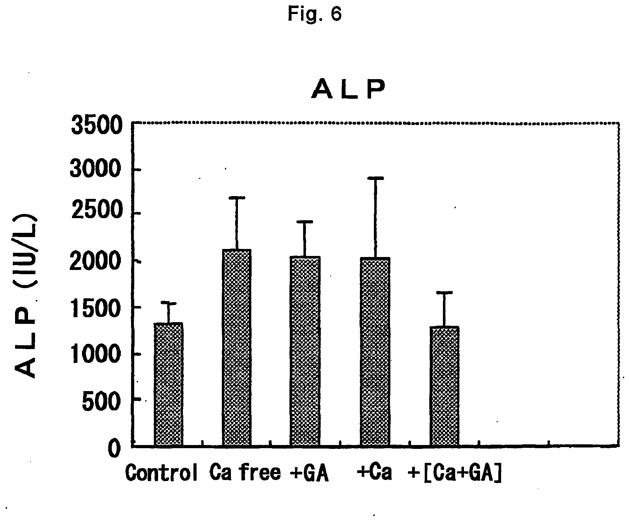

The results were shown in Figures 3-6.

The [Ca + GA]-treated group showed an increase of the body weight when the body weights 30 days after the administration were compared with that of the start (Figure 3). Hardness of the femur measured 30 days after the start of administration demonstrated the tendency of increasing hardness in the bone of [Ca + GA]-treated group compared with the Ca-treated group or the control group.

Furthermore, contents of various minerals, i.e. Ca, Zn, Mg and P in bone were measured, and the decrease of the Ca, Mg and P contents in bone of the [CA + GA]-treated group was significantly reduced. Alkaline phosphatase (ALP) is an enzyme to accelerate bone absorption of calcium and known to be increased in blood when the contents of calcium in bone is decreased, and also in this experiment the elevation of ALP in blood was observed in the Ca-treated group. However, coadministration of GA showed a tendency to reduce ALP elevation compared with the Ca-treated group.

These experimental results showed that coadministration of Ca and GA improved the efficiency of calcium absorption from the small intestine, and long-term administration reduced the decrease of calcium content in bone. Thus, a novel use of GA for an accelerating agent of bone absorption has been suggested.

### Preparation of a Drink Agent

The GA (10 g) is dispersed in warm water (40°C, 30 mL) with stirring. After cooling the mixture to 25°C, vitamin B1 (10 mg), vitamin B6 (10 mg), caffeine (50 mg), sugar (5 g), honey (5 g), citric acid (400 mg), sodium citrate (50 mg) and sodium benzoate (35 mg) are added with stirring. The pH value of the resulting mixture is adjusted to 6.0 by addition of lactic acid and/or 0.1N sodium hydroxide. Then the total volume of the resulting solution is adjusted to 5 ml by the addition of water.

GA used in this Example is purified as follows: GA obtained from Acacia Senegal in Sudan is powdered and dissolved in water. Then the resulting solution is filtered and the filtrate is spray-dried to give the said GA.

### INDUSTRIAL APPLICABILITY

An accelerating agent of calcium absorption provided by the present invention can be used in a field of calcium-enriched food for example, by mixing with various foods and drinks as well as combining itself with a calcium agent.

## Claims

1. An agent for use in the treatment or prevention of calcium deficiency by accelerating calcium absorption comprising Gum Arabic as an active ingredient **characterized in that** 1-100 g/day of said Gum Arabic is taken.

2. The agent for use according to claim 1, **characterized in that** 10-50 g/day of said Gum Arabic is taken.

3. The agent for use according to claim 1, **characterized in that** 10-20 g/day of said Gum Arabic is taken.

4. A calcium-enriched food comprising a calcium agent and the agent for use according to claims 1 to 3.

5. A drink agent to compensate for the lack of calcium comprising the agent for use according to claims 1 to 3.

6. Use of Gum Arabic in the manufacture of an agent for the treatment or prevention of: chronic renal failure or a calcium deficiency associated with chronic renal failure or cardiovascular disorders, including one or more of atherosclerosis, coronary artery disease, ischemic heart disease, hyperlipidemia and hypertension, or malaria or ulcerative colitis or gingivitis or dental plaque or learning defects such as Alzheimer's disease and senile dementia or osteoporosis or osteomalacia by accelerating calcium absorption **characterized in that** 1-100 g/day of said Gum Arabic is taken.

7. Use of claim 6, **characterized in that** 10-50 g/day of said Gum Arabic is taken.

8. Use of claim 6, **characterized in that** 10-20 g/day of said Gum Arabic is taken.

9. Use of claims 6 to 8, **characterized in that** the agent is a drink agent.

10. Use of Gum Arabic in the manufacture of an agent for reduction of cholesterol and the treatment of a hypercholesterolemic subject by accelerating calcium absorption **characterized in that** the agent additionally comprises a calcium agent and 1-100 g/day of said Gum Arabic is taken.

11. Use of claim 10, **characterized in that** 10-50 g/day of said Gum Arabic is taken.

12. Use of claim 10, **characterized in that** 10-20 g/day of said Gum Arabic is taken.

13. Use of claims 10 to 12, **characterized in that** the agent is a drink agent.

## Patentansprüche

1. Mittel zum Gebrauch in der Behandlung oder Vorbeugung von Kalziummangel durch Beschleunigung der Kalziumresorption, Gummi arabicum als Wirkstoff enthaltend, **dadurch gekennzeichnet, dass** 1-100 g/Tag des genannten Gummi arabicum eingenommen werden.

2. Mittel zum Gebrauch nach Patentanspruch 1, **dadurch gekennzeichnet, dass** 10-50 g/Tag des genannten Gummi arabicum eingenommen werden.

3. Mittel zum Gebrauch nach Patentanspruch 1, **dadurch gekennzeichnet, dass** 10-20 g/Tag des genannten Gummi arabicum eingenommen werden.

4. Mit Kalzium angereichertes Lebensmittel, ein Kalziummittel und das Mittel zum Gebrauch nach Patentansprüchen 1 bis 3 enthaltend.

5. Trinkbares Mittel zur Kompensation von Kalziummangel, das Mittel zum Gebrauch nach Patentansprüchen 1 bis 3 enthaltend.

6. Gebrauch von Gummi arabicum in der Herstellung eines Mittels zur Behandlung oder Vorbeugung von: chronischer Niereninsuffizienz oder Kalziummangel in Verbindung mit chronischer Niereninsuffizienz oder Herz-Kreislauferkrankungen, einschließlich einer oder mehrerer unter: Arteriosklerose, koronare Herzkrankheit, ischämische Herzkrankheit, Hyperlipidämie und Hypertonie, oder Malaria oder Colitis ulcerosa oder Gingivitis oder Zahnbelag oder Lernstörungen, wie Alzheimer-Krankheit oder senile Demenz oder Osteoporose oder Osteomalazie durch Beschleunigung der Kalziumresorption, **dadurch gekennzeichnet, dass** 1-100 g/Tag des genannten Gummi arabicum eingenommen werden.

7. Gebrauch nach Patentanspruch 6, **dadurch gekennzeichnet, dass** 10-50 g/Tag des genannten Gummi arabicum eingenommen werden.

8. Gebrauch nach Patentanspruch 6, **dadurch gekennzeichnet, dass** 10-20 g/Tag des genannten Gummi arabicum eingenommen werden.

9. Gebrauch nach den Patentansprüchen 6 bis 8, **dadurch gekennzeichnet, dass** das Mittel ein trinkbares Mittel ist.

10. Gebrauch von Gummi arabicum in der Herstellung eines Mittels zu Verringerung von Cholesterin und zur Behandlung eines Patienten mit Hypercholesterinämie durch Beschleunigung der Kalziumresorption, **dadurch gekennzeichnet, dass** das Mittel zusätzlich ein Kalziummittel enthält und 1-100 g/Tag des genannten Gummi arabicum eingenommen werden.

11. Gebrauch nach Patentanspruch 10, **dadurch gekennzeichnet, dass** 10-50 g/Tag des genannten Gummi arabicum eingenommen werden.

12. Gebrauch nach Patentanspruch 10, **dadurch gekennzeichnet, dass** 10-20 g/Tag des genannten Gummi arabicum eingenommen werden.

13. Gebrauch nach den Patentansprüchen 10 bis 12, **dadurch gekennzeichnet, dass** das Mittel ein trinkbares Mittel ist.

## Revendications

1. Agent destiné à être utilisé pour le traitement ou la prévention de la déficience en calcium en accélérant l'absorption de calcium comprenant de la gomme arabique comme principe actif, **caractérisé en ce qu'**il est pris 1 à 100 g/jour de ladite gomme arabique.

2. Agent destiné à être utilisé selon la revendication 1, **caractérisé en ce qu'**il est pris 10 à 50 g/jour de ladite gomme arabique.

3. Agent destiné à être utilisé selon la revendication 1, **caractérisé en ce qu'**il est pris 10 à 20 g/jour de ladite gomme arabique.

4. Denrées alimentaires enrichies en calcium comprenant un agent en calcium et l'agent destiné à être utilisé selon les revendications 1 à 3.

5. Agent de boisson pour compenser le manque de calcium comprenant l'agent destiné à être utilisé selon les revendications 1 à 3.

6. Utilisation de la gomme arabique pour la fabrication d'un agent pour le traitement ou la prévention d'insuffisance rénale chronique ou de déficience en calcium associée à une insuffisance rénale chronique ou à des troubles cardiovasculaires comprenant l'un ou plusieurs troubles parmi l'artériosclérose, les maladies coronariennes, la cardiopathie ischémique, l'hyperlipidémie et l'hypertension ou la malaria ou la rectocolite hémorragique ou la gingivite ou la plaque dentaire ou les difficultés d'apprentissage comme la maladie d'Alzheimer et la démence sénile ou l'ostéoporose ou l'ostéomalacie par l'accélération de l'absorption de calcium **caractérisée en ce qu'**il est pris 1 à 100 g/jour de ladite gomme arabique.

7. Utilisation selon la revendication 6, **caractérisée en ce qu'**il est pris 10 à 50 g/jour de ladite gomme arabique.

8. Utilisation selon la revendication 6, **caractérisée en ce qu'**il est pris 10 à 20 g/jour de ladite gomme arabique.

9. Utilisation selon les revendications 6 à 8, **caractérisée en ce que** l'agent est un agent de boisson.

10. Utilisation de la gomme arabique pour la fabrication d'un agent pour la réduction du cholestérol et le traitement d'un sujet hypercholestérolémique en accélérant l'absorption de calcium, **caractérisée en ce que** l'agent comprend de plus un agent en calcium et **en ce qu'**il est pris 1 à 100 g/jour de ladite gomme arabique.

11. Utilisation selon la revendication 10, **caractérisée en ce qu'**il est pris 10 à 50 g/jour de ladite gomme arabique.

12. Utilisation selon la revendication 10, **caractérisée en ce qu'**il est pris 10 à 20 g/jour de ladite gomme arabique.

13. Utilisation selon les revendications 10 à 12, **caractérisée en ce que** l'agent est un agent de boisson.
